(19) Europäisches Patentamt
European Patent Office
Office européen des brevets

(11) EP 1 743 627 B1

(12) **FASCICULE DE BREVET EUROPEEN**

(45) Date de publication et mention de la délivrance du brevet:
**17.12.2014 Bulletin 2014/51**

(51) Int Cl.:
***A61K 8/92*** *(2006.01)* ***A61K 8/81*** *(2006.01)*
***A61Q 1/06*** *(2006.01)*

(21) Numéro de dépôt: **06291032.8**

(22) Date de dépôt: **23.06.2006**

(54) **Composition de maquillage des lèvres de bonne tenue comprenant une résine de faible poids moléculaire**

Niedermolekulares Harz enthaltende, gute Beständigkeit zeigende Lippenschminkzusammensetzung

Highly resistant make up composition for the lips comprising a low molecular weight resin

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **13.07.2005 US 698790 P**

(43) Date de publication de la demande:
**17.01.2007 Bulletin 2007/03**

(73) Titulaire: **L'Oréal**
**75008 Paris (FR)**

(72) Inventeurs:
- **Blin, Xavier**
  **75015 Paris (FR)**
- **Lu, Shaoxiang**
  **Plainsboro, NJ 08536 (US)**
- **Bui, Hy Si**
  **Piscataway, NJ 08854 (US)**

(74) Mandataire: **Bonnet, Maiwenn et al**
**Cabinet Nony**
**3, rue de Penthièvre**
**75008 Paris (FR)**

(56) Documents cités:

| | |
|---|---|
| **EP-A- 0 850 649** | **EP-A- 1 469 042** |
| **EP-A- 1 621 229** | **EP-A2- 1 044 674** |
| **WO-A-99/38487** | **WO-A1-2005/058274** |
| **DE-A1- 19 707 309** | **FR-A1- 2 795 309** |
| **GB-A- 1 604 551** | **US-A- 4 699 780** |
| **US-A- 5 800 816** | **US-A1- 2001 007 654** |

- **DATABASE WPI Week 197736 Derwent Publications Ltd., London, GB; AN 1977-64148Y XP002405074 & JP 52 090637 A (SHISEIDO CO LTD) 30 juillet 1977 (1977-07-30)**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 743 627 B1

## Description

La présente invention se rapporte à une composition et à un procédé de maquillage des lèvres.

Avec la couleur, la tenue est un paramètre clef des produits de maquillage. La tenue de la couleur et le non transfert sont en général des propriétés antagonistes avec celle de la brillance. Pour obtenir de la tenue et du non transfert, il en effet souvent utilisé des compositions comportant des volatils et encore mieux des polymères véhiculés dans ces compositions contenant un solvant ou une huile volatil. Cependant ces compositions ne sont pas brillantes, du fait de la présence d'huile volatile.

Il a donc été imaginé de réaliser des formules en deux gestes permettant d'améliorer la brillance et le confort de la composition par l'application d'un topcoat brillant gras au dessus de la composition filmogène colorée. Cependant le départ du topcoat est inévitable dans la journée, si bien que la tenue du maquillage en terme de couleur reste bonne mais la disparition du topcoat détruit la brillance dont il était à l'origine. L'intérêt de conserver le maquillage coloré est donc largement dégradé du fait de cette perte de brillance.

D'une manière inattendue, les inventeurs ont découvert que l'incorporation d'une résine particulière dans une composition permet d'obtenir de bonnes propriétés de tenue, en particulier de tenue à l'huile, sans nuire à la brillance.

La présente invention a donc pour but de proposer une autre voie de formulation pour une composition de maquillage des lèvres ayant de bonnes propriétés de tenue et qui résolve en tout ou partie les problèmes liés aux voies de formulation conventionnelles.

La présente invention a plus précisément pour objet une composition cosmétique de maquillage des lèvres comprenant au moins une résine choisie parmi les résines hydrocarbonées et leurs mélanges, ladite résine présentant une masse moléculaire moyenne en nombre inférieure ou égale à 10 000 g/mol. La composition est avantageusement apte à former un film présentant une tenue à l'huile supérieure ou égale à 50 %, caractérisée en ce que la résine hydrocarbonée est choisie parmi:

- les polymères indéniques issus de la polymérisation en proportion majoritaire de monomère indène et en proportion minoritaire de monomères choisis parmi le styrène, le méthylindène, le méthylstyrène et leurs mélanges,
- les résines de pentadiène et d'indène, qui sont issues de la polymérisation d'un mélange de monomères de pentadiène et d'indène,

ladite composition comprenant une phase grasse liquide et étant caractérisée en ce qu'elle comprend en outre un copolymère séquencé hydrocarboné, soluble dans ladite phase grasse liquide et ledit copolymère étant en une quantité comprise entre 0,1 et 25 % en poids, du poids de la composition,
ledit copolymère séquencé hydrocarboné étant choisi parmi les copolymères séquencés comprenant au moins un bloc styrène et au moins un bloc comprenant des motifs choisis parmi le butadiène, l'éthylène, le propylène, le butylène, l'isoprène ou un de leurs mélanges.

Ladite composition est avantageusement apte à former un film présentant un dépôt dont la tenue à l'huile est supérieure ou égale à 50%, par exemple 55%, 60%, voir même 65%.

La tenue à l'huile peut être mesurée selon la méthode suivante.

On prépare trois lames de verre chacune recouverte d'une feuille de collagène comme suit.
A une température de 28°C, on prépare une feuille de collagène (Boyau artificiel naturin ep.0,06 mm, a. 0,10 mm, d. 120mm) de 5 cm par 10 cm de côté et on la met à conditionner au moins deux heures à 90% d'Humidité Relative (HR). On remet la feuille de collagène à l'air libre et on la fixe immédiatement fermement et entièrement sur une lame de verre 4,6 cm par 7,6 cm. On attache la feuille de collagène sur l'envers de la lame avec du scotch 3M. La surface du collagène doit être plane et exempte de pli. Chaque lame est laissée en conditions ambiantes pendant 24 heures, avant de réaliser le test.

On coupe un rectangle de 5 cm par 10 cm dans une assiette de Styrofoam (type Amoco Selectables Plastics DL Tableware) au moyen d'un couteau et d'une règle en suivant le contour d'une lame de verre. On applique le produit selon l'invention sur chaque lame de verre et sur le rectangle de Styrofoam comme suit. On applique la composition à l'aide d'un applicateur mécanique de 25 $\mu$m (bar coater).
On laisse les plaques en conditions ambiantes pendant 24 heures.

On applique 3 gouttes d'huile d'olive (environ 0,075 g) étalées avec un pinceau sur chacune des trois lames recouvertes de collagène et de composition. On sèche l'excédent avec un papier Kimwipes et on laisse les lames 30 minutes à température ambiante. On coupe 3 disques de Styrofoam blanc de 4 cm de diamètre.
On attache fermement avec du double face le disque de Styrofoam blanc au bout d'une masse de 2kg et, en exerçant une pression de 175g/cm$^2$, on pose doucement le poids à la surface d'une plaque (côté produit) et on procède, en 3 à 5 secondes, à une rotation d'un tour et demi du poids sur lui-même et ce, en maintenant la pression initale. On remonte le poids et on récupère le disque de Styrofoam. La mesure est effectuée pour chaque lame de verre avec un disque de Styrofoam propre.

On mesure ensuite le pourcentage de réflectance :

o du dépôt de produit appliqué sur l'échantillon rectangulaire de Styrofoam (référencé A),
o du disque propre de Styrofoam blanc (référencé B)
o du disque décollé du poids après avoir appliqué la pression sur la lame enduite de produit cosmétique (référencé C)

La réflectance est mesurée sur une gamme de longueur d'onde comprise entre 400 et 700nm à l'aide d'un analyseur spectral (ouverture 25 mm de diamètre) avec un illuminant D65/10degrés. On choisit la longueur d'onde du minimum de réflectance pour le disque 'taché'. A cette longueur d'onde, on calcule la tenue selon l'équation

$$100 * (1 - [(C-B)/(A-B)] )$$

La tenue à l'huile est égale à la moyenne des trois mesures.
En outre, un dépôt de la composition selon l'invention peut avantageusement présenter une brillance élevée.
La brillance d'un dépôt de la composition selon l'invention peut être mesurée selon la méthode décrite ci-après.
Avantageusement, le produit selon l'invention peut posséder une brillance supérieure ou égale à 5, en particulier supérieure ou égale à 10, notamment supérieure ou égale à 15, plus particulièrement supérieure ou égale à 20, notamment supérieure ou égale à 25, voire de l'ordre de 30.
Par "brillance", on désigne la brillance telle qu'elle peut être mesurée par la méthode suivante, en utilisant un appareillage de type gonio-réflectomètre comme par exemple le GRM-2000, commercialisé par la société MICROMODULES.
Les paramètres retenus pour cet appareillage sont les suivants :

- angle d'éclairage : 120°
- angle de détection : 60°
- angle de début : 50°
- angle de fin : 95°

On constitue un support de type mousse de forme rectangulaire, de dimensions 40 x 70 mm à partir d'une mousse de couleur brique, par exemple une mousse de néoprène de 3 mm d'épaisseur et qui possède une face adhésive, notamment une mousse connue sous la référence commerciale RE40x70 EP3 commercialisée par la société Joint Technique Lyonnais Ind.
On fixe, sur la face opposée à la face adhésive de ce support, un sparadrap transparent commercialisé par la société 3M® sous la référence commerciale BLENDERM® FH 5000-55113, présentant une qualité d'usage telle que l'application d'un rouge à lèvres sur ce revêtement est similaire à celle que l'on réalise sur les lèvres.
Le support mousse revêtu du sparadrap transparent est ensuite fixé, par collage, à l'aide de sa face à adhésive à une plaque métallique de dimension 40 x 70 mm. L'ensemble constitué par le support collé sur la plaque métallique forme une éprouvette.
L'opérateur réalise au total 5 éprouvettes identiques à celle décrite ci-dessus.
On va maintenant décrire un mode de mise en oeuvre du procédé d'évaluation de la brillance.
L'opérateur dispose l'éprouvette sur une plaque chauffante réglée à température de 38,5 °C, par exemple une plaque chauffante de type N81076 commercialisée par la société FISHER BIOBLOCK, et attend que la face du support portant le revêtement adhésif atteigne une température de 33 °C ± 1 °C.
Une fois que le support est à la température voulue, l'opérateur applique un film d'une épaisseur d'environ 15 μm de la composition.
La composition, qui est par exemple un rouge à lèvres, a été stocké à 24 °C ± 2 °C.
Le geste effectué par l'opérateur pour déposer le film de produit consiste en un aller/retour, de manière à obtenir un dépôt homogène. L'application du produit sur le support est effectuée de préférence de manière à être la plus représentative possible des conditions réelles d'application du produit. Le même produit à tester est appliqué de façon identique sur les cinq mêmes éprouvettes préparées précédemment.
On laisse sécher le film du produit, l'éprouvette étant placée sur la plaque chauffante, de telle sorte que le support reste à 33 °C ± 1 °C pendant 10 minutes.
On mesure la brillance du film du produit pour chacune des cinq éprouvettes.
A partir des valeurs mesurées, on établit la valeur moyenne de la brillance selon les conventions suivantes :

$$\overline{Brillance} = \frac{1}{N}\sum_i Brillance_i$$

écart-type :

$$\text{écart-type : } \sigma_{Brillance} = \sqrt{\frac{N\sum_i Brillance_i^2 - (\sum_i Brillance_i)^2}{N(N-1)}}$$

intervalle de confiance à 95 % :

$$\text{Brillance} \pm 1{,}96\sqrt{\frac{\sigma_{Brillance}}{N}}$$

où N désigne le nombre de mesures, c'est-à-dire 5 dans le cas d'espèce.

L'invention a aussi pour objet l'utilisation d'au moins une résine choisie parmi les résines hydrocarbonées et leurs mélanges, ladite résine présentant une masse moléculaire moyenne en nombre inférieure ou égale à 10 000 g/mol, dans une composition de maquillage des lèvres. La dite une composition est avantageusement apte à former un film présentant une tenue à l'huile supérieure ou égale à 50%, caractérisée en ce que la résine hydrocarbonée est choisie parmi:

- les polymères indéniques issus de la polymérisation en proportion majoritaire de monomère indène et en proportion minoritaire de monomères choisis parmi le styrène, le méthylindène, le méthylstyrène et leurs mélanges,
- les résines de pentadiène et d'indène, qui sont issues de la polymérisation d'un mélange de monomères de pentadiène et d'indène,

ladite composition comprenant une phase grasse liquide et étant caractérisée en ce qu'elle comprend en outre un copolymère séquencé hydrocarboné, soluble dans ladite phase grasse liquide et ledit copolymère étant en une quantité comprise entre 0,1 et 25 % en poids, du poids de la composition,
ledit copolymère séquencé hydrocarboné étant choisi parmi les copolymères séquencés comprenant au moins un bloc styrène et au moins un bloc comprenant des motifs choisis parmi le butadiène, l'éthylène, le propylène, le butylène, l'isoprène ou un de leurs mélanges.

La présente invention a en outre pour objet un procédé de maquillage des lèvres, dans lequel on applique sur les lèvres une composition telle que définie précédemment.

L'expression "au moins un", signifie un ou plusieurs composés individuels, ainsi que leurs mélanges.

La composition selon l'invention comprend un milieu physiologiquement acceptable, notamment un milieu cosmétiquement acceptable, c'est-à-dire un milieu compatible avec les fibres kératiniques.

## Résine

La résine utilisée dans la composition selon l'invention a de préférence une masse moléculaire moyenne en nombre inférieure ou égale à 10 000 g/mol, notamment allant de 250 à 10 000 g/mol de préférence inférieure ou égale à 5 000 g/mol, notamment allant de 250 à 5 000 g/mol, mieux, inférieure ou égale à 2 000 notamment allant de 250 à 2 000 g/mol et encore mieux inférieure ou égale à 1 000 g/mol notamment allant de 250 à 1 000 g/mol.

On détermine les masses molaires moyennes en nombre (Mn) par chromatographie liquide par perméation de gel (solvant THF, courbe d'étalonnage établie avec des étalons de polystyrène linéaire, détecteur réfractométrique).

La résine de la composition est avantageusement une résine dite tackifiante. De telles résines sont notamment décrites dans le Handbook of Pressure Sensitive Adhesive, edited by Donatas Satas, 3rd ed., 1989, p. 609-619.

La résine de la composition peut être choisie parmi la colophane ou un de ses dérivés, les résines hydrocarbonées et leurs mélanges.

Par « hydrocarboné », on entend un composé comprenant majoritairement du carbone et de l'hydrogène, et éventuellement des hétéroatomes tels que l'oxygène, l'azote ou le soufre. Le composé hydrocarboné est de préférence

constitué de carbone et d'hydrogène.

La colophane est un mélange comprenant majoritairement des acides organiques appelés acides de colophane (principalement des acides de type abiétique et de type pimarique).

Il existe trois types de colophane : la colophane ("gum rosin") obtenue par incision sur les arbres vivants, la colophane de bois ("wood rosin") qui est extraite des souches ou du bois de pins, et l'huile de tall ("tall oil rosin") qui est obtenue d'un sous-produit provenant de la production du papier.

Les dérivés de colophane peuvent être issus en particulier de la polymérisation, de l'hydrogénation et/ou de l'estérification (par exemple avec des alcools polyhydriques tels que l'ethylène glycol, le glycérol, le pentaérhytritol) des acides de colophanes. On peut citer par exemple les esters de colophanes commercialisés sous la référence FORAL 85, PENTALYN H et STAYBELITE ESTER 10 par la société HERCULES ; SYLVATAC 95 et ZONESTER 85 par la société ARIZONA CHEMICAL ou encore UNIREZ 3013 par la société UNION CAMP.

Les résines hydrocarbonées peuvent être choisies parmi les polymères oléfiniques de faible masse moléculaire qui peuvent être classifiées, selon le type de monomère, qu'elles comprennent, en les polymères indéniques, les résines de pentadiène, les résines des dimères du cyclopentadiène et les résines terpéniques.

Les polymères indéniques selon l'invention sont choisis parmi les polymères issus de la polymérisation en proportion majoritaire de monomère indène et en proportion minoritaire de monomères choisis parmi le styrène, le méthylindène, le méthylstyrène et leurs mélanges. Ces polymères peuvent éventuellement être hydrogénés, et peuvent présenter un poids moléculaire allant de 200 à 1 500 g/mol.

Le polymère hydrocarboné indénique est de préférence un copolymère bloc obtenu à partir d'indène, et de styrène ou d'un dérivé du styrène.

Selon un mode de réalisation selon l'invention, la résine est choisie parmi les résines indéniques, en particulier les copolymères indène/méthylstyrène/styrène hydrogéné commercialisées sous la dénomination « REGALITE » par la société Eastman Chemical, tels que la REGALITE R 1100, REGALITE R 1090, REGALITE R-7100, REGALITE R1010 HYDROCARBON RESIN, REGALITE R1125 HYDROCARBON RESIN.

Comme exemples de polymères indéniques, on peut citer ceux commercialisés sous la référence ESCOREZ 7105 par la société Exxon Chem., NEVCHEM 100 et NEVEX 100 par la société Neville Chem., NORSOLENE S105 par la société Sartomer, PICCO 6100 par la société Hercules et RESINALL par la société Resinall Corp.

Comme autre exemple de polymère indénique, on peut citer les résines de pentadiène et d'indène, qui sont issues de la polymérisation d'un mélange de monomères de pentadiène et d'indène tels que ceux décrits ci-dessus, comme par exemple les résines commercialisées sous la référence ESCOREZ 2101 par la société Exxon Chemicals., NEVPENE 9500 par la société Neville Chem., HERCOTAC 1148 par la société Hercules., NORSOLENE A 100 par la société Sartomer, WINGTACK 86, WINGTACK EXTRA et WINGTACK PLUS par la société Goodyear.

Les résines de pentadiène peuvent être choisies parmi celles issues de la polymérisation majoritairement du monomère 1,3-pentadiène (trans ou cis pipérylène) et de monomères minoritaires choisis parmi l'isoprène, le butène, le 2-méthyl-2-butène, le pentène, le 1,4-pentadiène et leurs mélanges. Ces résines peuvent présenter un poids moléculaire allant de 1 000 à 2 500 g/mol.

De telles résines du 1,3-pentadiène sont commercialisées par exemple sous les références PICCOTAC 95 par la société Eastman Chemical, ESCOREZ 1304 par la société Exxon Chemicals., NEVTAC 100 par la société Neville Chem. ou WINGTACK 95 par la société Goodyear.

Les résines des dimères du cyclopentanediène peuvent être choisies parmi celles issues de la polymérisation de premiers monomères choisis parmi l'indène et le styrène, et de deuxièmes monomères choisis parmi les dimères du cyclopentanediène tels que le dicyclopentadiène, le méthyldicyclopentadiène, les autres dimères du pentanediène, et leurs mélanges. Ces résines présentent généralement un poids moléculaire allant de 500 à 800 g/mol, telles que par exemple celles commercialisées sous la référence BETAPRENE BR 100 par la société Arizona Chemical Co., NEVILLE LX-685-125 et NEVILLE LX-1000 par la société Neville Chem., PICCODIENE 2215 par la société Hercules, PETRO-REZ 200 par la société Lawter ou RESINALL 760 par la société Resinall Corp.

Les résines terpéniques peuvent être choisies parmi celles issues de la polymérisation d'au moins un monomère choisi parmi l'$\alpha$-pinène, le $\beta$-pinène, le limonène, et leurs mélanges. Ces résines peuvent présenter un poids moléculaire allant de 300 à 2 000 g/mol. De telles résines sont commercialisées par exemple sous la dénomination PICCOLYTE A115 et S125 par la société Hercules, ZONAREZ 7100 ou ZONATAC 105 LITE par la société ARIZONA Chem.

On peut également citer comme résine hydrocarbonée, certaines résines commercialisées sous la dénomination EASTOTAC C6-C20 POLYOLEFINE par la société Eastman Chemical Co., sous la référence ESCOREZ 5300 par la société Exxon Chemicals ou encore les résines NEVILLAC HARD ou NEVROZ proposées par la société Neville Chem., les résines PICCOFYN A-100, PICCOTEX 100 ou PICCOVAR AP25 proposées par la société Hercules ou la résine SP-553 proposée par société Schenectady Chemical Co.

La résine peut être présente dans la composition selon l'invention en une teneur allant de 0,1 à 40% en poids par rapport au poids total de la composition, de préférence de 0,5 à 30% en poids et mieux de 1 à 20% en poids, par exemple de 2 à 15% en poids.

La résine peut être dotée d'au moins une température de transition vitreuse de préférence supérieure ou égale à 20°C, de préférence supérieure ou égale à 30°C, de préférence de l'ordre de 40°C. La température de transition vitreuse est avantageusement comprise entre 20°C et 300°C, par exemple entre 30°C et 100°C.

Les températures de transition vitreuse indiquées dans la présente demande peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\varpi_i / Tg_i) ,$$

$\overline{\omega}_i$ étant la fraction massique du monomère i dans la séquence considerée et $Tg_i$ étant la température de transition vitreuse de l'homopolymère du monomère i.

La mesure de la température de transition vitreuse (Tg) peut être effectuée selon la norme ASTM D3418-97, par analyse enthalpique différentielle (DSC "Differential Scanning Calorimetry") sur calorimètre, sur une plage de température comprise entre -100°C et +150°C à une vitesse de chauffe de 10°C/min dans des creusets en aluminium de 150 µl.

__Phase grasse liquide__

La composition selon l'invention comprend une phase grasse liquide à température ambiante (25°C) et pression atmosphérique (760 mm de Hg), composée d'un ou plusieurs corps gras non aqueux liquides à température ambiante, appelés aussi huiles ou solvants organiques, compatibles entre eux.

L'huile peut être choisie parmi les huiles volatiles et/ou les huiles non volatiles, et leurs mélanges.

Par " huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg). Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité $\leq$ 8 centistokes (8 $10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I) :

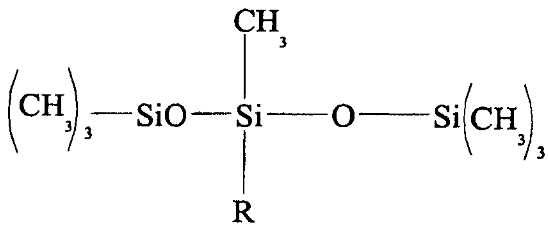

où R représente un groupe alkyle comprenant de 2 à 4 atomes de carbone et dont un ou plusieurs atomes d'hydrogène peuvent être substitués par un atome de fluor ou de chlore. Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des solvants volatils fluorés tels que le nonafluorométhoxybutane ou le perfluorométhylcyclopentane.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- et leurs mélanges.

Selon un mode de réalisation la composition contient une huile polaire, par exemple un alcool choisi parmi les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Selon un mode de réalisation, la phase grasse contient une huile ester. Cette huile ester peut être choisie parmi les esters des acides monocarboxyliques avec les monoalcools et polyalcools.

Avantageusement, ledit ester répond à la formule (I) suivante :

$$R_1\text{-CO-O-}R_2 \qquad (I)$$

où $R_1$ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

$R_2$ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par « éventuellement substitué », on entend que $R_1$ et ou $R_2$ peuvent porter un ou plusieurs substituants choisis, par exemple, parmi les groupements comprenant un ou plusieurs hétéroatomes choisi parmi O, N et S, tels que amino, amine, alcoxy, hydroxyle.

De préférence, le nombre total d'atomes de carbone de $R_1 + R_2$ est $\geq 9$.

$R_1$ peut représenter le reste d'un acide gras, de préférence supérieur, linéaire ou, de préférence ramifié comprenant de 1 à 40 et mieux de 7 à 19 atomes de carbone et $R_2$ peut représenter une chaîne hydrocarbonée linéaire ou de préférence ramifiée contenant de 1 à 40, de préférence de 3 à 30 et mieux de 3 à 20 atomes de carbone. De nouveau, de préférence, le nombre d'atomes de carbone de $R_1 + R_2 \geq 9$.

Des exemples des groupes $R_1$ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, éléostéarique, arachidonique, érucique, et de leurs mélanges.

Des exemples d'esters sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Avantageusement, les esters sont choisis parmi les composés de la formule (1) ci-dessus, dans laquelle $R_1$ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et $R_2$ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, $R_1$ est un groupe alkyle ramifié non substitué de 4 à 14 atomes de carbone, de préférence de 8 à 10 atomes de carbone et $R_2$ est un groupe alkyle ramifié non substitué de 5 à 15 atomes de carbone, de préférence de 9 à 11 atomes de carbone. De préférence dans la formule (I), $R_1$-CO- et $R_2$ ont le même nombre d'atomes de carbone et dérivent du même radical, de préférence alkyle ramifié non substitué, par exemple isononyle, c'est-à-dire que avantageusement la molécule d'huile ester est symétrique.

L'huile ester sera choisie, de préférence, parmi les composés suivants :

- l'isononanoate d'isononyle,
- l'octanoate de cétostéaryle,
- le myristate d'isopropyle,
- le palmitate d'éthyl-2-hexyle,
- le stéarate d'octyl 2-dodécyle,
- l'érucate d'octyl 2-dodécyle,
- l'isostéarate d'isostéaryle.

La phase grasse liquide peut représenter de 0,5 à 90 % en poids par rapport au poids total de la composition, de préférence de 1 à 60 % et de façon encore plus préférée de 2 à 40 % en poids.

## Copolymère bloc hydrocarboné

La composition selon l'invention contient outre la résine, un copolymère séquencé hydrocarboné appelé également copolymère bloc soluble dans une phase grasse liquide telle que définie précédemment.

Le copolymère peut présenter au moins un bloc dont la température de transition vitreuse, est de préférence inférieure à 20°C, de préférence inférieure ou égale à 0°C, de préférence inférieure ou égale à -20°C, de préférence encore inférieure ou égale à -40°C. La température de transition vitreuse dudit bloc peut être comprise entre -150°C et 20°C, notamment entre -100°C et 0°C.

Dans ce cas, lorsque la résine est dotée d'au moins une température de transition vitreuse, l'écart entre les températures de transition vitreuse de la résine et du copolymère est généralement supérieur à 20°C, de préférence supérieur à 40°C, et mieux supérieur à 60°C.

Lorsque la résine est dotée d'au moins une température de transition vitreuse, le copolymère séquencé est avantageusement un plastifiant de la résine décrite précédemment. On entend par plastifiant de la résine, un composé, qui associé

en quantité suffisante à la résine, abaisse la température de transition vitreuse de la résine telle que définie précédemment. Le composé plastifiant abaisse notamment la température de transition vitreuse du polymère d'au moins 2, 3 ou 4 °C, de préférence de 5°C à 20°C. Dans un mode de réalisation préféré, le composé plastifiant abaisse notamment la température de transition vitreuse du polymère d'au moins 2, 3 ou 4 °C, de préférence de 5°C à 20°C.

Le copolymère bloc est choisi parmi les copolymères séquencés, hydrocarbonés éventuellement hydrogénés, dibloc, tribloc, multi-bloc ou radiaux, et leurs mélanges.

Les copolymères séquencés hydrocarbonés comprennent au moins un bloc styrène et au moins un bloc comprenant des motifs choisis parmi le butadiène, l'éthylène, le propylène, le butylène, l'isoprène ou un de leurs mélanges.

Comme copolymère dibloc, on peut citer les copolymères de styrène/éthylène-propylène (constitués d'un bloc styrène et d'un bloc obtenu à partir d'éthylène et de propylène), les copolymères de styrène/éthylène-butylène, les copolymères styrène/butadiène, les copolymères styrène/isoprène. De tels copolymères sont notamment vendus sous la dénomination Kraton® G1701E par la société Kraton Polymers.

Comme copolymère tribloc, on peut citer les copolymères de styrène/éthylène-propylène/styrène, les copolymères de styrène/éthylène-butylène/styrène, les copolymères de styrène/éthylène-butadiène/styrène, les copolymères de styrène/isoprène/styrène, les copolymères de styrène/butadiène/styrène. Des polymères triblocs sont notamment vendus sous les dénominations Kraton® G1650, Kraton® G1652, Kraton® D1101, Kraton® D1102, Kraton® D1160 par la société Kraton Polymers.

On peut notamment utiliser comme copolymère bloc, le mélange d'un copolymère dibloc et d'un copolymère tribloc. Dans ce mode de réalisation, le copolymère dibloc et le copolymère tribloc sont choisis parmi les copolymères séquencés comprenant au moins un bloc styrène et au moins un bloc comprenant des motifs choisis parmi le butadiène, l'éthylène, le propylène, le butylène, l'isoprène.

On peut citer à titre d'exemple le produit commercialisé sous la référence Kraton G 1657 M, qui est un mélange de copolymère dibloc styrène/éthylène-butylène, et de copolymère tribloc styrène/éthylène-butylène/styrène dans les proportions 30/70, la température de transition vitreuse du bloc éthylène-butylène étant égale à -60°C environ.

On peut également utiliser un mélange de copolymère hydrogéné tribloc styrène/butylène-éthylène/styrène et de polymère étoile hydrogéné éthylène/propylène/styrène. De tels mélanges sont par exemple vendus par la société PENRECO sous les dénominations commerciales VERSAGEL® M5960 et VERSAGEL® M5670.

Le copolymère bloc hydrocarboné est présent en une quantité comprise entre 0,1 et 25% en poids du poids de la composition, notamment de 0,5 à 15 % en poids.

Le ratio massique entre la résine hydrocarbonée et le copolymère bloc hydrocarboné est de préférence compris entre 80/20 et 40/60, notamment entre 75/25 et 50/50.

La composition peut comprendre une phase aqueuse, qui comprend de l'eau et/ou au moins un solvant hydrosoluble.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions selon l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$ et $C_4$ et les aldéhydes en $C_2$-$C_4$.

La phase aqueuse (eau et éventuellement le solvant miscible à l'eau) peut être présente, en une teneur allant de 5 % à 95 % en poids, par rapport au poids total de la composition, de préférence allant de 10 % à 80 % en poids, et préférentiellement allant de 15 % à 60 % en poids.

La composition selon l'invention peut contenir des agents tensioactifs émulsionnants présents notamment en une proportion allant de 0,1 à 30 % en poids par rapport au poids total de la composition, mieux de 1 à 15 % et mieux de 2 à 10 %.

La composition selon l'invention peut comprendre au moins un agent structurant de la phase huileuse choisi parmi

- les polymères semi-cristallins, décrits par exemple dans le document EP 1396259,
- les gélifiants lipophiles minéraux, comme les argiles éventuellement modifiées comme les hectorites modifiées par un chlorure d'ammonium d'acide gras en $C_{10}$ à $C_{22}$, comme l'hectorite modifiée par du chlorure de di-stéaryl diméthyl ammonium telle que, par exemple, celle commercialisée sous la dénomination de Bentone 38V® par la société ELEMENTIS. On peut également citer la silice pyrogénée éventuellement traitée hydrophobe en surface dont la taille des particules est inférieure à 1 $\mu$m.
- les organogélateurs moléculaires notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.
- les organopolysiloxanes élastomériques partiellement ou totalement réticulés, de structure tridimensionnelle, comme

ceux commercialisés sous les dénominations de KSG6®, KSG16® et de KSG18® par la société SHIN-ETSU

- les polycondensats de type polyamide comprenant au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ;
- les polyamides siliconés du type polyorganosiloxane tels que ceux décrits dans les documents US-A-5 874 069, US-A-5,919,441, US-A-6,051,216 et US-A-5,981,680 comme par exemple ceux commercialisés sous la référence Dow Corning 2-8179 Gellant par la société DOW CORNING;
- les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges ;

La composition selon l'invention comprend avantageusement au moins une cire.

La cire considérée est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55°C.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeilles, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone, les cires fluorées.

La composition selon l'invention peut comprendre une teneur en cires allant de 5 à 20 % en poids par rapport au poids total de la composition, en particulier elle peut en contenir de 7 à 15 %.

La composition peut comprendre, outre la résine, un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

La composition selon l'invention peut également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

La composition selon l'invention peut en outre comprendre au moins une charge.

Les charges peuvent être choisies parmi celles bien connues de l'homme du métier et couramment utilisées dans les

compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société Atochem, de poly-β-alanine et de polyéthylène, les poudres de polymères de tétrafluoroéthylène comme le Téflon®, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.
On peut également utiliser un composé susceptibles de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel®007WU par la Société AKZO NOBEL.
Les charges peuvent représenter de 0,1 à 25 %, en particulier de 1 à 20 % en poids par rapport au poids total de la composition.
La composition de l'invention peut comprendre, en outre, tout additif usuellement utilisé en cosmétique tels que les antioxydants, les conservateurs, les fibres, les parfums, les neutralisants, les gélifiants, les épaississants, les vitamines, les agents de coalescence, les plastifiants, et leurs mélanges.

Comme actifs cosmétiques pouvant être utilisés dans les compositions selon l'invention, on peut citer notamment des antioxydants, les conservateurs, les parfums, les neutralisants, émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

Les compositions selon l'invention peuvent être préparées selon des méthodes connues de l'homme du métier.

La composition selon l'invention peut être conditionnée dans un récipient délimitant au moins un compartiment qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Les exemples qui suivent sont présentés à titre illustratif et non limitatif de l'invention.

## <u>Exemple</u>

On prépare le rouge à lèvres suivant selon l'invention.
Les quantités sont données en gramme.

| | |
|---|---|
| Copolymère styrène/méthyl styrène/indène hydrogéné (REGALITE R1100 de Eastmann) | 16 |
| Hydrogenated styrene/butdiene copolymer (Kraton 1657 M) * | 10 |

(suite)

| | |
|---|---|
| Isododécane | Qsp 100 |
| Octyldodécanol | 3,1 |
| Pigments | 3,1 |
| Nacres | 2 |

* Le Kraton 1657 M est un mélange de copolymère dibloc et de copolymère tribloc dans les proportions 70/30 comprenant des blocs styrène et des blocs éthylène-butylène.

On a mesuré la tenue à l'huile et la brillance de la composition selon les méthodes de mesure indiquées précédemment dans la description.
La tenue à l'huile, mesurée selon la méthode décrite précédemment est égale à 68%.

<u>Mode opératoire:</u>

1. On réalise un broyat pigmentaire des pigments dans la phase huileuse en effectuant 3 passages du mélange à la broyeuse tri cylindres.
2. On introduit dans un poêlon, le ou les copolymères et l'huile, on place le mélange sous agitation Rayneri à une température de 100°C.
3. quand on observe un mélange liquide transparent, on introduit le broyat et les nacres, on maintient place le mélange sous agitation Rayneri à une température de 100°C pendant 20 minutes
4. On réalise un qsp 100% avec l'huile
5. On coule la formule dans des pots étanches à l'isododécane.

## Revendications

**1.** Composition cosmétique de maquillage des lèvres comprenant au moins une résine hydrocarbonée, ladite résine présentant une masse moléculaire moyenne en nombre inférieure ou égale à 10 000 g/mol, ladite composition étant apte à former un film présentant une tenue à l'huile supérieure ou égal à 50 %, **caractérisée en ce que** la résine hydrocarbonée est choisie parmi :

- les polymères indéniques issus de la polymérisation en proportion majoritaire de monomère indène et en proportion minoritaire de monomères choisis parmi le styrène, le méthylindène, le méthylstyrène et leurs mélanges, et
- les résines de pentadiène et d'indène, qui sont issues de la polymérisation d'un mélange de monomères de pentadiène et d'indène,

ladite composition comprenant une phase grasse liquide et étant **caractérisée en ce qu'**elle comprend en outre un copolymère séquencé hydrocarboné, soluble dans ladite phase grasse liquide et ledit copolymère étant en une quantité comprise entre 0,1 et 25 % en poids, du poids de la composition,
ledit copolymère séquencé hydrocarboné étant choisi parmi les copolymères séquencés comprenant au moins un bloc styrène et au moins un bloc comprenant des motifs choisis parmi le butadiène, l'éthylène, le propylène, le butylène, l'isoprène ou un de leurs mélanges.

**2.** Composition selon la revendication 1, **caractérisée en ce qu'**elle est apte à former un film présentant une tenue à l'huile supérieure ou égale à 55 %, voire même supérieure ou égale à 60 %, et mieux encore supérieure ou égale à 65 %.

**3.** Composition selon la revendication 1 ou 2, **caractérisée en ce que** la résine présente une masse moléculaire moyenne en nombre allant de 250 à 10 000 g/mol de préférence inférieure ou égale à 5 000 g/mol, notamment allant de 250 à 5 000 g/mol, mieux, inférieure ou égale à 2 000, notamment allant de 250 à 2 000 g/mol et encore mieux inférieure ou égale à 1 000 g/mol, notamment allant de 250 à 1 000 g/mol.

4. Composition selon l'une des revendications précédentes, **caractérisée en ce que** la résine ou le polymère hydrocarboné indénique est choisi parmi les copolymères indène/méthylstyrène/styrène hydrogéné.

5. Composition selon l'une quelconque des revendications précédentes, **caractérisée en ce que** la résine est présente en une teneur allant de 0,1 à 20 % en poids par rapport au poids total de la composition, de préférence de 0,5 à 15 % en poids et mieux de 1 à 10 % en poids.

6. Composition selon l'une quelconque des revendications précédentes, **caractérisée en ce que** la phase grasse liquide comprend au moins une huile volatile hydrocarbonée.

7. Composition selon l'une quelconque des revendications précédentes, **caractérisée en ce que** la phase grasse liquide comprend un alcool gras.

8. Composition selon l'une quelconque des revendications précédentes, **caractérisée en ce que** la phase grasse liquide représente 0,5 à 90 % en poids par rapport au poids total de la composition, de préférence de 1 à 60 % et de façon encore plus préférée de 2 à 40 % en poids.

9. Composition selon l'une quelconque des revendications précédentes, **caractérisée en ce que** le copolymère séquencé hydrocarboné est présent en une quantité comprise entre 0,5 et 15 % en poids du poids de la composition.

10. Composition selon la revendication précédente, **caractérisée en ce que** le ratio massique entre la résine hydrocarbonée et le copolymère séquencé hydrocarboné est compris entre 80/20 et 40/60, notamment entre 75/25 et 50/50.

11. Composition selon l'une quelconque des revendications précédentes, **caractérisée en ce qu'**elle comprend une cire, un gélifiant minéral ou un organogélateur.

12. Composition selon la revendication 11 **caractérisée en ce que** la cire est présente en une teneur allant de 5 à 20 % en poids par rapport au poids total de la composition, en particulier elle peut en contenir de 7 à 15 %.

13. Composition selon l'une des revendications précédentes, **caractérisée en ce qu'**elle comprend une matière colorante.

14. Composition selon la revendication 13, **caractérisée en ce que** la matière colorante représente de 0,01 à 30 % en poids par rapport au poids total de la composition.

15. Procédé de maquillage lèvres, **caractérisé en ce que** l'on applique sur lèvres, une composition telle que définie selon l'une quelconque des revendications 1 à 14.

16. Utilisation d'au moins une résine hydrocarbonée de masse moléculaire moyenne en nombre inférieure ou égale à 10 000 g/mol, dans une composition de maquillage des lèvres pour obtenir une composition apte à former un film présentant une tenue à l'huile supérieure ou égale à 50%, **caractérisée en ce que** la résine hydrocarbonée est choisie parmi:

    - les polymères indéniques issus de la polymérisation en proportion majoritaire de monomère indène et en proportion minoritaire de monomères choisis parmi le styrène, le méthylindène, le méthylstyrène et leurs mélanges,
    - les résines de pentadiène et d'indène, qui sont issues de la polymérisation d'un mélange de monomères de pentadiène et d'indène,

    ladite composition comprenant une phase grasse liquide et étant **caractérisée en ce qu'**elle comprend en outre un copolymère séquencé hydrocarboné, soluble dans ladite phase grasse liquide et ledit copolymère étant en une quantité comprise entre 0,1 et 25 % en poids, du poids de la composition
    ledit copolymère séquencé hydrocarboné étant choisi parmi les copolymères séquencés comprenant au moins un bloc styrène et au moins un bloc comprenant des motifs choisis parmi le butadiène, l'éthylène, le propylène, le butylène, l'isoprène ou un de leurs mélanges.

**Patentansprüche**

1. Kosmetische Lippen-Make-Up-Zusammensetzung enthaltend wenigstens ein Kohlenwasserstoffharz, das eine zahlenmittlere Molekularmasse von höchstens 10000 g/mol aufweist, wobei die Zusammensetzung dazu geeignet ist, einen Film mit einem Ölgehalt von wenigstens 50% zu bilden, **dadurch gekennzeichnet, dass** das Kohlenwasserstoffharz ausgewählt ist aus:

    - Indenpolymeren, die aus der Polymerisation einer überwiegenden Menge an Indenmonomeren und einer Mindermenge von Monomeren, ausgewählt aus Styrol, Methlyinden, Methylstyrol und deren Gemischen hervorgeht, und
    - Harzen aus Pentadien und Inden, die hervorgehen aus der Polymerisation eines Gemischs von Monomeren von Pentadien und Inden,

    wobei die Zusammensetzung eine flüssige Fettphase enthält und **dadurch gekennzeichnet ist, dass** sie des weiteren ein Kohlenwasserstoffblockcopolymer enthält, das in der genannten flüssigen Fettphase löslich ist und wobei das Copolymer in einer Menge im Bereich von 0,1 bis 25 Gew.-%, bezogen auf das Gewicht der Zusammensetzung, enthalten ist,
    wobei das Kohlenwasserstoffblockcopolymer ausgewählt ist aus den Block-copolymeren, die wenigstens einen Styrolblock und wenigstens einen Block enthalten, der Einheiten aufweist ausgewählt aus Butadien, Ethylen, Propylen, Butylen, Isopren oder deren Gemischen.

2. Zusammensetzung gemäß Patentanspruch 1, **dadurch gekennzeichnet, dass** sie geeigent ist, einen Film zu bilden, der einen Ölgehalt von mnindestens 55%, insbesondere mindestens 60% und noch besser mindestens 65% aufweist.

3. Zusammensetzung gemäß Patentanspruch 1 oder 2, **dadurch gekennzeichnet, dass** das Harz eine zahlenmittlere Molekularmasse von 250-10000 g/mol, bevorzugt höchstens 5000 g/mol, insbesondere 250 bis 5000 g/mol, besser höchstens 2000, insbesondere 250 bis 2000 g/mol und noch besser höchstens 1000 g/mol, insbesondere 250 bis 1000 g/mol aufweist.

4. Zusammensetzung gemäß einem der vorangehenden Patentansprüche, **dadurch gekennzeichnet, dass** das Harz oder das indenische Kohlenwasserstoffpolymer ausgewählt ist aus den hydrierten Copolymeren Inden/Methlystyrol/Styrol.

5. Zusammensetzung gemäß einem der vorangehenden Patentansprüche, **dadurch gekennzeichnet, dass** das Harz in einer Menge von 0,1 bis 20 Gew.-% enthalten ist, bezogen auf das Gesamtgewicht der Zusammensetzung, bevorzugt von 0,5 bis 15 Gew.-% und besser von 1 bis 10 Gew.-%.

6. Zusammensetzung gemäß einem der vorangehenden Patentansprüche, **dadurch gekennzeichnet, dass** flüssige Fettphase mindestens ein flüchtiges Kohlenwasserstofföl enthält.

7. Zusammensetzung gemäß einem der vorangehenden Patentansprüche, **dadurch gekennzeichnet, dass** die flüssige Fettphase einen Fettalkohol enthält.

8. Zusammensetzung gemäß einem der vorangehenden Patentansprüche, **dadurch gekennzeichnet, dass** die flüssige Fettphase 0,5 bis 90 Gew.-% bezogen auf das Gesamtgewicht der Zusammensetzung darstellt, bevorzugt 1 bis 60 % und noch stärker bevorzugt 2 bis 40 Gew.-%.

9. Zusammensetzung gemäß einem der vorangehenden Patentansprüche, **dadurch gekennzeichnet, dass** das Kohlenwasserstoffblockcopolymer in einer Menge von 0,5 bis 15 Gew.-%, bezogen auf das Gewicht der Zusammensetzung enthalten ist.

10. Zusammensetzung gemäß einem der vorangehenden Patentansprüche, **dadurch gekennzeichnet, dass** das Massenverhältnis von Kohlenwasserstoffharz zu Kohlenwasserstoffblockcopolymer im Bereich von 80/20 bis 40/60 liegt, insbesondere von 75/25 bis 50/50.

11. Zusammensetzung gemäß einem der vorangehenden Patentansprüche, **dadurch gekennzeichnet, dass** sie ein Wachs, ein gelbildendes Mineral oder einen organischen Gelbildner enthält.

12. Zusammensetzung gemäß Patentanspruch 11, **dadurch gekennzeichnet, dass** das Wachs in einem Gehalt von 5 bis 20 Gew.-%, bezogen auf das Gesamtgewicht der Zusammensetzung vorliegt, insbesondere, kann sie davon 7 bis 15 % enthalten.

13. Zusammensetzung gemäß einem der der vorangehenden Patentansprüche, **dadurch gekennzeichnet, dass** sie einen Farbstoff enthält.

14. Zusammensetzung gemäß Patentanspruch 13, **dadurch gekennzeichnet, dass** der Farbstoff von 0,01 bis 30 Gew.-%, bezogen auf das Gesamtgewicht der Zusammensetzung ausmacht.

15. Verfahren zum Lippen-Make-Up, **dadurch gekennzeichnet, dass** auf die Lippen eine Zusammensetzung wie in einem der Patentansprüche 1 bis 14 definiert aufgetragen wird.

16. Verwendung von wenigstens einem Kohlenwasserstoffharz, der zahlenmittleren Molekularmasse von höchstens 10000 g/mol in einer Lippen-Make-Up-Zusammensetzung, um eine Zusammensetzung zu erhalten, die geeignet ist, einen Film mit einem Ölgehalt von mindestens 50% zu bilden, **dadurch gekennzeichnet, dass** das Kohlenwasserstoffharz ausgewählt ist aus:

    - Indenpolymeren, gebildet aus der Polymerisation einer überwiegenden Menge an Indenmonomeren und einer Mindermenge von Monomeren, ausgewählt aus Styrol, Methlyinden, Methylstyrol und deren Gemischen,
    - Harze von Pentadien und Inden, die erhalten sind durch die Polymerisation eines Gemischs von Monomeren von Pentadien und Inden,

    wobei die Zusammensetzung eine flüssige Fettphase enthält und **dadurch gekennzeichnet ist, dass** sie des weiteren ein Kohlenwasserstoffblockcopolymer enthält, das in der genannten flüssigen Fettphase löslich ist und wobei das Copolymer in einer Menge von 0,1 bis 25 Gew.-% bezogen auf das Gewicht der Zusammensetzung vorliegt, wobei das Kohlenwasserstoffblockcopolymer wenigstens einen Styrol Block und wenigstens einen Block aufweist, der Einheiten ausgewählt aus Butadien, Ethylen, Propylen, Butylen, Isopren oder deren Gemischen enthält.

## Claims

1. A cosmetic lip makeup composition comprising at least one hydrocarbon-based resin, the said resin having a number average molecular weight equal to or less than 10 000 g/mol, the said composition being capable of forming a film having resistance to oil equal to or higher than 50 %, **characterized in that** the hydrocarbon-based resin is chosen from among:

    - indene polymers derived from polymerization in majority proportion of indene monomer and minority proportion of monomers chosen from among styrene, methylindene, methylstyrene and the mixtures thereof; and
    - resins of pentadiene and indene, derived from polymerization of a mixture of pentadiene and indene monomers,

    the said composition comprising a liquid fatty phase and being **characterized in that** it further comprises a hydrocarbon-based block copolymer soluble in the said liquid fatty phase, and the said copolymer being in an amount of between 0.1 and 25 % by weight of the weight of the composition,
    the said hydrocarbon-based block copolymer being chosen from among the block copolymers comprising at least one styrene block and at least one block comprising repeat units chosen from among butadiene, ethylene, propylene, butylene, isoprene or one of the mixtures thereof.

2. The composition according to claim 1, **characterized in that** it is capable of forming a film having resistance to oil equal to or higher than 55 %, even equal to or higher than 60 % and further preferably equal to or higher than 65 %.

3. The composition according to claim 1 or 2, **characterized in that** the resin has a number average molecular weight ranging from 250 to 10 000 g/mol, preferably equal to or lower than 5 000 g/mol, more preferably 250 to 5 000 g/mol, further preferably equal to or lower than 2 000, in particular ranging from 250 to 2 000 g/mol and more particularly equal to or lower than 1000 g/mol, further particularly ranging from 250 to 1 000 g/mol.

4. The composition according to one of the preceding claims, **characterized in that** the resin or indene hydrocarbon-based polymer is chosen from among the indene/methylstyrene/hydrogenated styrene copolymers.

5. The composition according to any of the preceding claims, **characterized in that** the resin content ranges from 0.1 to 20 % by weight relative to the total weight of the composition, preferably 0.5 to 15 % by weight, and better still from 1 to 10 % by weight.

6. The composition according to any of the preceding claims, **characterized in that** the liquid fatty phase comprises at least one hydrocarbon-based volatile oil.

7. The composition according to any of the preceding claims, **characterized in that** the liquid fatty phase comprises a fatty alcohol.

8. The composition according to any of the preceding claims **characterized in that** the liquid fatty phase represents 0.5 to 90 % by weight relative to the total weight of the composition, preferably 1 to 60 % and further preferably 2 to 40 % by weight.

9. The composition according to any of the preceding claims, **characterized in that** the hydrocarbon-based block copolymer is contained in an amount of between 0.5 and 15 % by weight of the weight of the composition.

10. The composition according to the preceding claim, **characterized in that** the weight ratio between the hydrocarbon-based resin and the hydrocarbon-based block copolymer is between 80:20 and 40:60, in particular between 75:25 and 50:50.

11. The composition according to any of the preceding claims, **characterized in that** it comprises a wax, a mineral gelling agent or an organo-gelling agent.

12. The composition according to claim 11, **characterized in that** the wax content ranges from 5 to 20 % by weight relative to the total weight of the composition, in particular it may contain 7 to 15 % thereof.

13. The composition according to one of the preceding claims, **characterized in that** it comprises a dyestuff.

14. The composition according to claim 13, **characterized in that** the dyestuff represents 0.01 to 30 % by weight relative to the total weight of the composition.

15. A method for making up the lips, **characterized in that** a composition such as defined in any of claims 1 to 14 is applied to the lips.

16. The use of at least one hydrocarbon-based resin of number average molecular weight equal to or lower than 10 000 g/mol in a lip makeup composition to obtain a composition capable of forming a film having resistance to oil equal to or higher than 50 %, **characterized in that** the hydrocarbon-based resin is chosen from among:

    - indene polymers derived from polymerization in majority proportion of indene monomer and minority proportion of monomers chosen from among styrene, methylindene, methystyrene and the mixtures thereof;
    - resins of pentadiene and indene derived from the polymerization of a mixture of pentadiene and indene monomers,

    the said composition comprising a liquid fatty phase and being **characterized in that** it further comprises a hydrocarbon-based block copolymer soluble in the said liquid fatty phase, and the said copolymer being in an amount of between 0.1 and 25 % by weight of the weight of the composition
    the said hydrocarbon-based block copolymer being chosen from among block copolymers comprising at least one styrene block and at least one block comprising repeat units chosen from among butadiene, ethylene, propylene, butylene, isoprene or one of the mixtures thereof.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 847752 A
- EP 1396259 A
- EP 1068854 A
- EP 1086945 A
- WO 0247031 A
- US 5874069 A
- US 5919441 A
- US 6051216 A
- US 5981680 A
- US 4887622 A
- FR 2796529
- FR 2761959
- FR 2792618


### Littérature non-brevet citée dans la description


- Handbook of Pressure Sensitive Adhesive. 1989, 609-619
- Polymer Handbook. John Wiley, 1989
- Specialist Surfactants. 1997, 209-263